# EUROPEAN PATENT SPECIFICATION

(11) **EP 3 104 871 B1**
(45) Date of publication and mention of the grant of the patent: **14.03.2018**
(21) Application number: 15704781.2
(22) Date of filing: 12.02.2015
(51) Int. Cl.: A61K 38/14, A61K 9/00, A61K 47/14, A61K 31/575, A61K 47/12, A61K 38/12

(54) **TOPICAL COMPOSITION COMPRISING AN ANTIBACTERIAL AGENT**
TOPISCHE ZUSAMMENSETZUNG ENTHALTEND EINE ANTIBAKTERIELLE SUBSTANZ
COMPOSITION TOPIQUE COMPRENANT UN AGENT ANTIBACTÉRIEN

(30) Priority: 12.02.2014 GB 201402448
(43) Date of publication of application: 21.12.2016
(73) Proprietor: Buzzz Pharmaceuticals Limited, Dublin 5 (IE)
(72) Inventor: GREEN, Darren M., Cardiff CF3 2PX (GB); WALTERS, Kenneth A., Cardiff CF3 2PX (GB)
(74) Representative: HGF Limited
(86) International application number: PCT/EP2015/052998
(87) International publication number: WO 2015/121363

(56) References cited:
- WO-A1-2012/103037
- WO-A2-2006/081518
- WO-A2-2008/018085
- US-A1- 2004 081 681
- FLORIAN WEIS: "Daptomycin: a novel lipopeptide antibiotic against Gram-positive pathogens", INFECTION AND DRUG RESISTANCE, 1 August 2010 (2010-08-01), page 95, XP055176062, DOI: 10.2147/IDR.S6961

## Description

### FIELD OF THE INVENTION

The present invention relates to a novel formulation. More specifically, the present invention relates to novel antibiotic formulations that are suitable for topical administration. The formulations of the present invention are suitable for the topical treatment of bacterial infections on the skin and/or mucosal surfaces, such as, for example, the topical treatment of Methicillin-resistant *Staphylococcus aureus* (MRSA), and other complicated skin and skin structure infections (cSSSIs), such as, for example, gangrene.

The present invention therefore also relates to these formulations for use in the treatment of topical infections (for example MRSA), as well as to processes for the preparation of the formulations of the invention.

### BACKGROUND OF THE INVENTION

Methicillin-resistant *Staphylococcus aureus* (MRSA) is a bacterium responsible for several problematic and difficult-to-treat infections in humans. It is also called oxacillin-resistant *Staphylococcus aureus* (ORSA). MRSA is any strain of *Staphylococcus aureus* that has developed, through the process of natural selection, resistance to beta-lactam antibiotics, which include the penicillins (methicillin, dicloxacillin, nafcillin, oxacillin, etc.) and the cephalosporins. Strains unable to resist these antibiotics are classified as methicillin-sensitive *Staphylococcus aureus*, or MSSA. The evolution of such resistance does not cause the organism to be more intrinsically virulent than strains of *Staphylococcus aureus* that have no antibiotic resistance, but resistance does make MRSA infection more difficult to treat with standard types of antibiotics, and therefore more dangerous.

MRSA is especially troublesome in hospitals and nursing homes, where patients with open wounds, invasive devices, and weakened immune systems are at greater risk of infection than the general public.

*Staphylococcus aureus* is a bacteria found on the skin and in the nostrils of approximately 30% of healthy people. A subset of this population carry the MRSA strain *of Staphylococcus aureus* on their skin or in their nostrils and are unaware, because it does them no harm and they have no symptoms. This is known as '*colonisation*'. MRSA colonisation tends to occur in the nose, under the arms (axilla), the groin, or perineum (skin between the rectum and genitals).

MRSA can cause particular harm if it is able to enter the body. For example, MRSA can cause serious wound infections, chest infections or infections of the blood stream. As such, MRSA infection is a significant risk factor in major surgery.

Many hospitals now screen patients for MRSA prior to admittance for elective surgical procedures. If a patient is identified as a carrier of MRSA on either their skin or in their nose then a treatment plan can be implemented to eradicate the MRSA prior to the surgery.

As the areas of MRSA colonisation on the skin are typically the groin, the perineal area and the axilla regions, these regions are notoriously difficult to treat using traditional creams and ointments. In particular, it can be hard to get good surface coverage of these areas of the body with a traditional "manually applied" antibiotic formulation.

There is also a need for improved antibiotic formulations to treat other topical infections, such as, for example, impetigo and complicated skin and skin structure infections (cSSSIs). An example of a cSSSI is an infection such as gangrene.

There is therefore a need for improved antibiotic formulations for topical application to eradicate MRSA and other pathogenic bacteria.

In particular, there is a need for a formulation that can be applied to the desired area of the body.

There is also a need for a formulation that can provide better coverage (and therefore treatment) of the infected area, including poorly accessible areas such as the groin, the perineum and axilla regions of the body.

One antibiotic compound that can be used for the treatment of MSRA infection is daptomycin. Daptomycin is a lipopeptide antibiotic that has the structural formula I shown below:

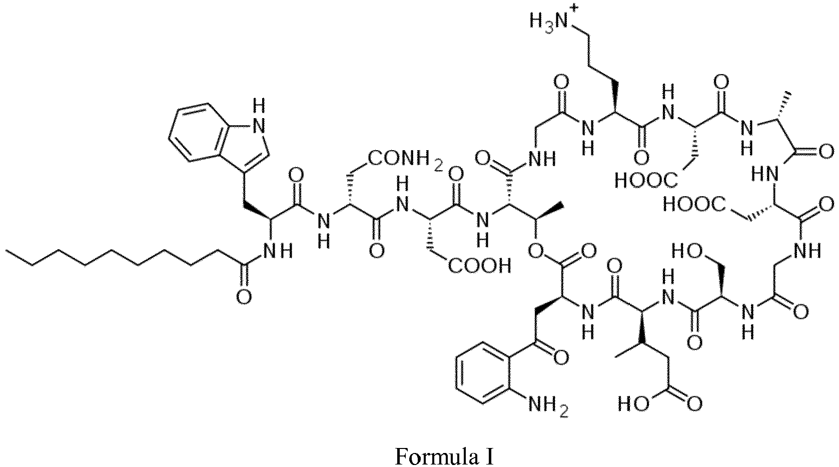

Daptomycin is described in US Patent No. 4,537,717, the entire contents of which are incorporated herein by reference.

In addition to its use for the treatment of MRSA, daptomycin is also used for the treatment of methicillin-susceptible *Staphylococcus aureus* (MSSA); streptococcal infections such as *Streptococcus pyrogenes, Streptococcus agalactiae, Streptococcus dysgalactiae*; and *Enterococcus faecalis*. Daptomycin is commercially available as an intravenous infusion (Cubicin™), but no topical formulations of daptomycin are available.

Daptomycin is stable in a lyophilised form, but it is susceptible to hydrolysis in aqueous environments.

There is therefore a particular need for improved formulations of daptomycin that are: (i) suitable for topical administration; and (ii) stable.

### SUMMARY OF THE INVENTION

The present invention provides a novel pharmaceutical composition that is suitable for topical application for the treatment of skin infections, such as, for example, MRSA. Thus, in a first aspect the invention provides a pharmaceutical composition suitable for topical application to the body, said composition comprising daptomycin or a pharmaceutically acceptable salt thereof that is dissolved and/or suspended within an oily vehicle, wherein the daptomycin or pharmaceutically acceptable salt thereof is present in an amount of 0.01 wt.% to 10 wt.% of the total composition, and wherein the moisture content of the composition is less than 1 wt.%. The pharmaceutical composition of the invention is further described in the present claims. In a further aspect, the invention also provides a pharmaceutical composition of the invention for use in the treatment of topical infections, optionally wherein said topical infection is selected from impetigo, MRSA or a complicated skin and/or skin structure infection (cSSSI), or in the eradication of topical MRSA prior to surgery. The invention also provides a method of preparing a pharmaceutical composition of the invention, said method comprising suspending daptomycin or a pharmaceutically acceptable salt thereof in an oily vehicle. The invention further provides a device comprising a pharmaceutical composition of the invention, wherein the device is configured to dispense a volume of the composition for topical application.

Described herein is a pharmaceutical composition suitable for topical application to the body, said composition comprising an antibacterial agent that is dissolved and/or suspended in an oily vehicle as defined herein.

Also described is a topical pharmaceutical composition comprising an antibacterial agent that is dissolved and/or suspended in an oily vehicle as defined herein.

As defined further herein, the oily vehicle is suitably a low viscosity fluid that is capable of spontaneously spreading on the skin or mucosal surfaces to which is it is applied. This means that it is not necessary to manually spread the composition on the skin or mucosal surface to which it is applied, although manual spreading may additionally be used if desired.

Thus, also described herein is a pharmaceutical composition suitable for topical application to the body, said composition comprising an antibacterial agent that is dissolved and/or suspended within an oily vehicle;
wherein the pharmaceutical composition is spontaneously spreadable upon application to a surface of the body.

Further described herein is a topical pharmaceutical composition comprising an antibacterial agent that is dissolved and/or suspended within an oily vehicle;
wherein the pharmaceutical composition is spontaneously spreadable upon application to a surface of the body.

Also described is a pharmaceutical composition suitable for topical application to the body, said composition comprising:
(i) an antibacterial agent;
(ii) an oily vehicle; and
(iii) optionally a suspending agent and/or a surfactant, wherein the suspending agent and/or surfactant renders the antibacterial agent dispersible in the oily vehicle;
and wherein the pharmaceutical composition is spontaneously spreadable upon application to the a surface of the body.

Also described herein is a topical pharmaceutical composition, said composition comprising:
(i) an antibacterial agent;
(ii) an oily vehicle; and
(iii) optionally a suspending agent and/or a surfactant, wherein the suspending agent and/or surfactant renders the antibacterial agent dispersible in the oily vehicle;
and wherein the pharmaceutical composition is spontaneously spreadable upon application to the a surface of the body.

Also described herein is a pharmaceutical composition as defined herein for use in the treatment of topical infections (e.g. infections present on the skin or accessible mucosal surfaces, such as the nasal passages).

Further described is a method of treating a topical infection, said method comprising administering to a human or animal subject in need of such treatment a therapeutically effective amount of a pharmaceutical composition as defined herein.

Also described is a pharmaceutical composition as defined herein for use in the treatment of MRSA, impetigo or other complicated skin and skin structure infections (cSSSIs).

Further described herein is a method of treating MRSA, impetigo or other complicated skin and skin structure infections (cSSSIs), said method comprising administering to a human or animal subject in need of such treatment a therapeutically effective amount of a pharmaceutical composition as defined herein.

Also described is a pharmaceutical composition as defined herein for use in the eradication of MRSA prior to surgery.

Further described herein is a method of eradicating MRSA prior to surgery, said method comprising administering to a human or animal subject in need of such treatment a therapeutically effective amount of a pharmaceutical composition as defined herein.

Also described herein is a method of preparing a pharmaceutical composition as defined herein, said method comprising suspending an antibacterial agent as defined herein in an oily vehicle.

Further described herein is a device comprising a pharmaceutical composition as defined hereinbefore, wherein the device is configured to dispense a volume of the composition (for example, in the form of a spray, jet, or one or more droplets or fixed bolus, optionally for application via some form of applicator utensil).

### DETAILED DESCRIPTION OF THE INVENTION

### Definitions

Unless otherwise stated, the following terms used in the specification and claims have the following meanings set out below.

The terms "treating" or "treatment" include prophylaxis as well as the alleviation of established symptoms of a disease or condition. "Treating" or "treatment" therefore includes: (1) preventing or delaying the appearance of clinical symptoms of the disease or condition developing in a subject that may be afflicted with or predisposed to the disease or condition, but does not yet experience or display clinical or subclinical symptoms of the disease or condition, (2) inhibiting the disease or condition, *i.e.*, arresting, reducing or delaying the development of the disease or condition or a relapse thereof (in case of maintenance treatment) or at least one clinical or subclinical symptom thereof, or (3) relieving or attenuating the disease or condition, *i.e.*, causing regression of the disease or condition or at least one of its clinical or subclinical symptoms.

The term "topical pharmaceutical composition" is used herein to mean a pharmaceutical composition adapted and suitable for topical application.

The term "topical application" means application to the body surfaces, including the skin, mucous membranes (e.g. the nasal passages) and any sites of injury or wounds present on the in the skin or mucous membranes.

The term "spontaneously spreadable" is used herein to define the pharmaceutical compositions of the present disclosure and means that, upon topical application (as defined above), the composition spreads spontaneously from the point of application. The oily vehicle is suitably a low viscosity fluid that spreads over the skin or mucosal surface following application. This means that it is not necessary to manually spread the composition on the skin or mucosal surface to which it is applied, although manual spreading may additionally be used if desired. Suitably, on contact with the skin/mucosa, the composition will spread across the surface of the skin/mucosa to cover an area that is at least three-fold larger, and more suitably five-fold larger, than the area covered by a droplet of water of equivalent volume. Suitably the spreading to cover an area that is at least three-fold larger, and more suitably five-fold larger, than the area covered by a droplet of water of equivalent volume occurs within a short time period of time, for example, up to 5 minutes, or more preferably up to 2 minutes.

The term "D90" is used herein to refer to the particle size distribution of antimicrobial agent particles. D90 is the value of the particle diameter at 90% in the cumulative distribion of particles sizes present. For example, if D90 = 50 µm, then 90% of the particles in the sample are smaller than 50 µm and 10% are larger than 50 µm.

### Pharmaceutical compositions

As previously stated, the present disclosure provides novel pharmaceutical compositions that are suitable for topical application to the body to treat infections of the skin and mucosal surfaces, such as, for example, MRSA.

In particular, described herein is a pharmaceutical composition suitable for topical application to the body, said composition comprising an antibacterial agent that is dissolved and/or suspended in an oily vehicle as defined herein.

Also described is a topical pharmaceutical composition comprising an antibacterial agent that is dissolved and/or suspended in an oily vehicle as defined herein.

Also described is a pharmaceutical composition suitable for topical application to the body, said composition comprising an antibacterial agent that is dissolved and/or suspended within an oily vehicle;
wherein the pharmaceutical composition is spontaneously spreadable upon application to a surface of the body.

Also described is a topical pharmaceutical composition comprising an antibacterial agent that is dissolved and/or suspended within an oily vehicle;
wherein the pharmaceutical composition is spontaneously spreadable upon application to a surface of the body.

Suitably, the antibacterial agent is suspended within the oily vehicle. Suitably, the antimicrobial agent is uniformly dispersed or dispersible within the oily vehicle. In this context, the terms "dispersed or dispersible" mean that the antimicrobial agent is either maintained in the form of a homogenous dispersion within the oily vehicle or that a homogenous dispersion can be easily attained by agitating the composition (e.g. shaking the composition prior to use).

Suitably, the composition is a liquid at room temperature and at skin temperature (34-38°C).

The pharmaceutical compositions of the present disclosure possess a number of advantageous properties.

Firstly, the use of an oily vehicle renders the compositions ideally suited to antimicrobial agents that are susceptible to hydrolysis in aqueous environments (such as, for example, daptomycin). Thus, commercially viable topical formulations with good stability can be prepared.

As indicated above, the antimicrobial agent is either uniformly dispersed in the oily vehicle or dispersible in the vehicle, so a homogenous suspension can be easily attained by agitating (e.g. shaking) the composition prior to use.

The spreadable nature of the pharmaceutical composition, which is primarily imparted by the nature of the oily vehicle, also enables the compositions to spontaneously spread across the desired portion of the body following application. This property of the compositions means that there is less need for a patient or carer to manually spread the formulation over the affected area of the body. This provides a number of advantages:
- There is a lower risk of the infection (e.g. MRSA) spreading if manual contact can be avoided or at least minimised.
- Controlled doses of the composition can be applied, for example as a spray or spot on application of a defined volume.
- The uniformity (and low viscosity) with which the composition covers the affected area of the body can be improved (manual spreading can inherently provide poor uniformity of coverage).
- Compared to traditional topical preparations, the compositions of the present disclosure can improve the treatment of infections that reside in poorly accessible part of the body, such as, for example, the groin, the perineal area and the axilla regions (which are a particular problem in the case of MRSA).

The formulations also exhibit good emolliency.

### Antimicrobial agent

The antimicrobial agent present in the pharmaceutical compositions of the present disclosure may be any antimicrobial agent suitable for topical use.

The compositions of the present disclosure are particularly suited to antimicrobial agents that have poor stability in aqueous media (but the scope of the present disclosure should not be construed as being limited to such antimicrobial agents).

The antimicrobial agents used in the compositions of the present disclosure will typically have little or no solubility in the oily vehicle of the composition. As such, the antimicrobial agent is present as a dispersion or suspension within the oily vehicle.

The antimicrobial agent may be present as a pharmaceutically acceptable salt or solvate. Accordingly, references herein to antimicrobial agents (either as generically or by reference to specifically named antimicrobial agent) will include, where appropriate, any pharmaceutically acceptable salts or solvates thereof.

In one instance, the antimicrobial agent is present as discrete particles.

Suitably, the antimicrobial agent is in a fine particulate form, suitably a microparticulate or nanoparticulate form.

Suitably, the particles of antimicrobial agent have a D90 of 75µm or less, more suitably a D90 of 60µm or less, more suitably D90 of 50µm or less, and even more suitably less than 40 µm or less.

The antimicrobial agent may be present at any suitable amount in the composition of the disclosure. Suitably, the antimicrobial agent is present in an amount of 0.01 wt.% to 10 wt.%, more suitably 0.05 wt.% to 5 wt.%, even more suitably 0.1 wt.% to 4 wt.%, even more suitably 0.2 wt.% to 4 wt.%, and most suitably 0.5 wt.% to 3 wt.%.

A person skilled in the art will be able to select appropriate antimicrobial agents for inclusion in the compositions of the present disclosure by virtue of their physicochemical properties and their potential use for the treatment of topical infections. Some examples of suitable antimicrobial agents include daptomycin, vancomycin, mupirocin, polymixin B, clindamycin, retapamulin, neomycin, bacitracin and fusidic acid, or pharmaceutically acceptable salts thereof.

In an instance, the pharmaceutical composition may comprise two or more antimicrobial agents, for example agents selected from those mentioned herein.

In an instance, an antimicrobial agent is selected from the group consisting of daptomycin, vancomycin, mupirocin, polymixin B, clindamycin, retapamulin, neomycin, bacitracin and fusidic acid or pharmaceutically acceptable salts thereof.

In a further instance, an antimicrobial agent is selected from daptomycin, vancomycin and/or mupirocin.

In a particular instance, and in the composition of the invention, the antimicrobial agent is daptomycin, or a pharmaceutically acceptable salt thereof. For the avoidance of doubt, and as previously stated above, references herein to daptomycin should be construed as covering daptomycin or any pharmaceutically acceptable salt or solvate thereof. Suitably, the average particle size of the daptomycin is less than 50 µm, more suitably less than 40 µm, and even more suitably less than 25 µm. Suitably, the daptomycin is present in an amount of 0.01 wt.% to 10 wt.% of the composition. more suitably 0.05 wt.% to 5 wt.%, even more suitably 0.1 wt.% to 4 wt.%, and most suitably 0.5 wt.% to 3 wt.%.

In a particular instance, the antimicrobial agent is daptomycin present at a concentration of 2 +/- 0.5 wt.% of the composition. In the composition of the invention, daptomycin or a pharmaceutically acceptable salt thereof is present in an amount of 0.01 wt.% to 10 wt.% of the total composition.

### Oily vehicle

The oily vehicle provides the medium in which the antimicrobial agent or agents is/are dissolved/suspended/dispersed. The oily vehicle also imparts the spreadability characteristics of the compositions of the present disclosure.

In instances where the antimicrobial agent is suspended or dispersed in the oily vehicle, any suitable oily vehicle in which the antimicrobial agent is insoluble or poorly soluble, and that is "spontaneously spreadable" as defined herein, may be used in the compositions of the present disclosure.

One or more oil components may be present in the oily vehicle. In an instance, the oily vehicle comprises one or more oil components selected from the group consisting of:
mono-, di- and/or triglycerides comprising glycerol and (6-18C) fatty acids;
propylene glycol esters of (6-18C) fatty acids;
(6-18C) fatty acid esters (e.g. isopropyl myristate);
(6-18C) fatty alcohols;
silicone fluids/oils (such as, for example dimethicone, hexamethyldisiloxane, octamethyltrisiloxane and cyclomethicone); and
lanolin/lanolin derivatives.

Particular examples of (6-18C) fatty acids include caprylic acid, capric acid, lauric acid, myristic acid, sebacic, and oleic acid.

In an instance, the fatty acid components present in the oily vehicle are (8-18C) fatty acids.

Particular examples of glycerides that may be used in the compositions of the present disclosure include glycerol monooleate (e.g. Capmul® GMO-50), glycerol monocaprylate/caprate (e.g. Capmul® MCM), and caprylic/capric triglyceride (e.g. Captex® 355).

Particular examples of propylene glycol esters of (6-18C)fatty acids include propylene glycol monocaprylate (Capyrol® 90) and propylene glycol dicaprylocaprate (Labrafac® PG).

Particular examples of (6-18C) fatty acid esters, include (8-18C) fatty acid (1-6C) alkyl esters, such as, for example, isopropyl myristate, dioctyladipate, isobutyl palmitate, octyldodecyl cocoate, and/or diisopropyl sebacate.

Particular examples of (6-18C) fatty alcohols include capric alcohol, lauryl alcohol, myristyl alcohol, cetyl alcohol, palmitoleyl alcohol and steryl alcohol.

Particular examples of silicone fluids/oils include dimethicone, hexamethyldisiloxane, octamethyltrisiloxane and cyclomethicone.

Particular examples of lanolin/lanolin derivatives that may be used in the compositions of the present disclosure include acetylated lanolin (e.g. Modulan®).

The oily vehicle typically forms the largest proportion of the compositions of the present disclosure. Typically, the oily vehicle will constitute between 70 wt.% and 99.5 wt.% of the composition. More typically, it will constitute 80 - 95 wt.% of the composition and even more typically between 85 and 93 wt.% of the composition.

In certain instances, the oily vehicle comprises one or more oil components selected from the group consisting of:
mono-, di- and/or triglycerides comprising glycerol and (6-18C) fatty acids;
propylene glycol esters of (6-18C) fatty acids;
(6-18C) fatty acid esters (e.g. isopropyl myristate); and
(6-18C) fatty alcohols.

In another instance, the oily vehicle comprises one or more oil components selected from the group consisting of:
mono-, di- and/or triglycerides comprising glycerol and (6-18C) fatty acids;
propylene glycol esters of (6-18C) fatty acids; and
(6-18C) fatty acid esters (e.g. isopropyl myristate).

In some further instances, it advantageous to add further oily emollients to the oily vehicle. The additional emollients may constitute up to 70% wt.% of the composition and more suitably up to 65 wt.% of the composition. Any suitable oily emollients may be used in the compositions. Examples of suitable emollient components include silicone fluids/oils (such as, for example dimethicone, hexamethyldisiloxane, octamethyltrisiloxane and cyclomethicone); lanolin/lanolin derivatives; cocoyl adipic acid / trimethylproprane copolymer, adipate fatty acid esters such as diisobutyl adipate, (6C fatty acid esters) fatty acid esters of citric acid, e.g. tributylcitrate, and volatile oils, e.g. peppermint oil.

In a particular instance, the oily vehicle comprises one or more oil components selected from the group consisting of:
mono-, di- and/or triglycerides comprising glycerol and (6-18C) fatty acids;
propylene glycol esters of (6-18C) fatty acids;
(6-18C) fatty acid esters (e.g. isopropyl myristate); and
silicone fluids/oils (such as, for example dimethicone, hexamethyldisiloxane and cyclomethicone).

In a further instance, the oily vehicle comprises (the ranges refer to the wt.% of the total pharmaceutical composition):
5 - 45 wt.% (6-18C) fatty acid esters (e.g. isopropyl myristate);
5 - 60 wt.% mono-, di- and/or triglycerides comprising glycerol and (6-18C) fatty acids;
0 - 10 wt.% propylene glycol esters of (6-18C) fatty acids;
0 - 70 wt.% silicone fluids/oils (such as, for example dimethicone, hexamethyldisiloxane, octamethyltrisiloxane and cyclomethicone); and
0 - 5 wt.% lanolin/lanolin derivative.

In a further instance, the oily vehicle comprises (the ranges refer to the wt.% of the total pharmaceutical composition):
10 - 20 wt.% (6-18C) fatty acid esters (e.g. isopropyl myristate);
10 - 30 wt.% mono-, di- and/or triglycerides comprising glycerol and (8-18C) fatty acids;
0 - 10 wt.% propylene glycol esters of (6-18C) fatty acids; and
0 - 70 wt.% silicone fluids/oils (such as, for example dimethicone, hexamethyldisiloxane, octamethyltrisiloxane and cyclomethicone).

In a further instance, the oily vehicle comprises (the ranges refer to the wt.% of the total pharmaceutical composition):
10 - 20 wt.% (6-18C) fatty acid esters (e.g. isopropyl myristate);
10 - 30 wt.% mono-, di- and/or triglycerides comprising glycerol and (6-18C) fatty acids;
0 - 10 wt.% propylene glycol esters of (6-18C) fatty acids; and
40 - 70 wt.% silicone fluids/oils (such as, for example dimethicone, hexamethyldisiloxane, octamethyltrisiloxane and cyclomethicone).

In a further instance, the oily vehicle comprises (the ranges refer to the wt.% of the total pharmaceutical composition):
10 - 20 wt.% (6-18C) fatty acid esters (e.g. isopropyl myristate);
10 - 20 wt.% mono-, di- and/or triglycerides comprising glycerol and (6-18C) fatty acids; and
40 - 70 wt.% silicone fluids/oils (such as, for example dimethicone, hexamethyldisiloxane, octamethyltrisiloxane and cyclomethicone).

In a further instance, the oily vehicle comprises (the ranges refer to the wt.% of the total pharmaceutical composition):
10 - 40 wt.% isopropyl myristate;
10 - 60 wt.% caprylic/capric triglyceride/medium chain triglycerides;
0-30 wt.% dimethicone;
0 - 40 wt.% hexamethyldisiloxane; and
0 - 30 wt.% cyclomethicone.

In a further instance, the oily vehicle comprises (the ranges refer to the wt.% of the total pharmaceutical composition):
10 - 20 wt.% isopropyl myristate;
10 - 20 wt.% caprylic/capric triglyceride/medium chain triglycerides;
10 - 20 wt.% dimethicone;
10 - 40 wt.% hexamethyldisiloxane; and
10 - 30 wt.% cyclomethicone.

### Suspending agent

In certain instances, the pharmaceutical composition further comprises a suspending agent. A suspending agent functions to help prevent the aggregation of the antibacterial agent particles suspended in the oily vehicle. Therefore, a suspending agent may be used to enable the antimicrobial particles to either remain homogenously dispersed within the oily vehicle over time or enable a homogenous dispersion to be easily attained by agitating (e.g. shaking) the composition.

The suspending agent suitably has little or no solubility in the oily vehicle.

Particularly suitable suspending agents include one or more of the following:
cross-linked polymers suitably in a micronized form, e.g PVP (crospovidone) & sodium croscarmellose;
clays: micronized clays aluminium phyllosilicate (bentonite);
magnesium/aluminium silicates;
silicas: silica (e.g. Syliod®, Cab-o-sil®), silicon dioxide (e.g. Aerosil®); and hydrogenated oils: trihydroxystearin (Thixcin® R)

In a particular instance, the suspending agent is micronized crospovidone (e.g. Kollidon® CL-M). This material also functions as a water scavenger, and thus may improve the stability of any water-sensitive components.

The suspending agent is present in an amount sufficient to suspend the antibacterial agent. Typically, the suspending agent will be present in an amount of 0.2 - 15 wt.%, more typically 0.2 - 10 wt.% and most typically at 0.2 - 7 wt.%.

### Surfactants

The oily vehicle may further comprise one or more oily surfactants. The amount of such oily surfactants present will typically be up to 7.5 wt.% of the total composition, more typically up to 5 wt.% and most typically up to 3 wt.%.

Such surfactants suitably have a HLB (hydrophilic-lipophilic balance) value of less than 7.

Examples of suitable surfactants include polyoxyethylated fatty acid (10-18C)esters of sorbitan (e.g. Span 20, Span 80); (8-18C) fatty acid esters, and polyoxypropylene / polyoxyethylene block co-polymers (Poloxamers®).

In certain instances, the pharmaceutical composition comprises a suspending agent as defined above and an oily surfactant as defined herein.

### Stabiliser

In certain instances, the composition may further comprise one or more stabilisers.

In instances where the pharmaceutical composition comprises daptomycin, a divalent metal salt, such as calcium and/or magnesium salts, may be added. Examples of suitable salts to add to the compositions are calcium and/or magnesium fatty acid salts (e.g. calcium and/or magnesium stearate), calcium and/or magnesium sulphate, calcium and/or magnesium trisilicate, and calcium and/or magnesium carbonate/ hydroxide In a particular instance, calcium stearate is used as the stabiliser.

The amount of stabiliser present may be 0 - 5 wt.% of the total composition, for example 0.5 to 3 wt.%.

### Moisture content

Suitably the moisture content of the compositions of the present disclosure is less than 5 wt.%, preferably less than 2 wt.%, more preferably less than 1 wt.%, and most preferably less than 0.5 wt.%. The moisture content of a composition of the invention is less than 1 wt.%.

The moisture content will typically arise as a consequence of the small amounts of moisture that may be present in certain excipients used to prepare the compositions.

### Particular instances of the disclosure

In an instance, the pharmaceutical composition comprises:
(i) 0.05 - 5 wt.% of an antibacterial agent;
(ii) 75 - 99.5 wt.% of an oily vehicle; and
(iii) optionally 0.5 - 10 wt.% of a suspending agent.

In a further instance, the pharmaceutical composition comprises:
(i) 1 - 3 wt.% of an antibacterial agent;
(ii) 80 - 98 wt.% of an oily vehicle; and
(iii) optionally 1 - 8 wt.% of a suspending agent.

In another instance, the pharmaceutical composition comprises:
(i) 1 - 3 wt.% of an antibacterial agent;
(ii) 80 - 96 wt.% of an oily vehicle; and
(iii) optionally 3 - 7 wt.% of a suspending agent.

In another instance, the pharmaceutical composition comprises:
0.05 - 5 wt.% of daptomycin or a pharmaceutically acceptable salt thereof;
5 - 45 wt.% (6-18C) fatty acid esters (e.g. isopropyl myristate);
5 - 60 wt.% mono-, di- and/or triglycerides comprising glycerol and (6-18C) fatty acids;
0 - 10 wt.% propylene glycol esters of (6-18C) fatty acids;
0 - 70 wt.% silicone oils (such as, for example dimethicone, hexamethyldisiloxane, octamethyltrisiloxane and cyclomethicone);
0 - 5 wt.% lanolin/lanolin derivative;
up to 7.5 wt.% of a surfactant selected from polyoxyethylated fatty acid (10-18C)esters of sorbitan (e.g. Span 20, Span 80); (8-18C) fatty acid esters, and polyoxypropylene / polyoxyethylene block co-polymers (Poloxamers®);
0.5 - 10 wt.% of a suspending agent selected from one or more of the following:
   cross-linked polymers suitably in a micronized for, e.g PVP (crospovidone) & sodium croscarmellose;
   clays: micronized clays aluminium phyllosilicate (bentonite);
   magnesium/aluminium silicates;
   silicas: silica (Syliod®, Cab-o-sil®), silicon dioxide (Aerosil®); and
   hydrogenated oils: trihydroxystearin (Thixcin® R); and
0 - 5 wt.% of a divalent metal salt.

In another instance, the pharmaceutical composition comprises:
1 - 5 wt.% of daptomycin or a pharmaceutically acceptable salt thereof;
5 - 45 wt.% (6-18C) fatty acid esters (e.g. isopropyl myristate);
5 - 60 wt.% mono-, di- and/or triglycerides comprising glycerol and (6-18C) fatty acids;
0 - 10 wt.% propylene glycol esters of (6-18C) fatty acids;
0 - 70 wt.% silicone oils (such as, for example dimethicone, hexamethyldisiloxane, octamethyltrisiloxane and cyclomethicone);
0 - 5 wt.% lanolin/lanolin derivative;
0 - 5 wt.% of a surfactant selected from polyoxyethylated fatty acid (10-18C)esters of sorbitan (e.g. Span 20, Span 80); (8-18C) fatty acid esters, and polyoxypropylene / polyoxyethylene block co-polymers (Poloxamers®);
1 - 8 wt.% of a suspending agent selected from one or more of the following:
   cross-linked polymers suitably in a micronized for, e.g PVP (crospovidone) & sodium croscarmellose;
   clays: micronized clays aluminium phyllosilicate (bentonite);
   magnesium/aluminium silicates;
   silicas: silica (Syliod®, Cab-o-sil®), silicon dioxide (Aerosil®); and
   hydrogenated oils: trihydroxystearin (Thixcin® R); and
1 - 3 wt.% of a divalent metal salt.

In another instance, the pharmaceutical composition comprises:
1 - 5 wt.% of daptomycin or a pharmaceutically acceptable salt thereof;
5 - 45 wt.% (6-18C) fatty acid esters (e.g. isopropyl myristate);
5 - 60 wt.% mono-, di- and/or triglycerides comprising glycerol and (8-18C) fatty acids;
0 - 70 wt.% silicone oils (such as, for example dimethicone, hexamethyldisiloxane, octamethyltrisiloxane and cyclomethicone);
1 - 5 wt.% of a surfactant selected from polyoxyethylated fatty acid (10-18C)esters of sorbitan (e.g. Span 20, Span 80); (8-18C) fatty acid esters, and polyoxypropylene / polyoxyethylene block co-polymers (Poloxamers®);
1 - 8 wt.% of a suspending agent selected from one or more of the following:
   cross-linked polymers suitably in a micronized for, e.g PVP (crospovidone) & sodium croscarmellose;
   clays: micronized clays aluminium phyllosilicate (bentonite);
   magnesium/aluminium silicates;
   silicas: silica (Syliod®, Cab-o-sil®), silicon dioxide (Aerosil®); and
   hydrogenated oils: trihydroxystearin (Thixcin® R); and
1 - 3 wt.% of a divalent calcium and/or magnesium salt.

In another instance, the pharmaceutical composition comprises:
1 - 5 wt.% of daptomycin or a pharmaceutically acceptable salt thereof;
10 - 20 wt.% (6-18C) fatty acid esters (e.g. isopropyl myristate);
10 - 20 wt.% mono-, di- and/or triglycerides comprising glycerol and (6-18C) fatty acids;
50 - 70 wt.% silicone oils (such as, for example dimethicone, hexamethyldisiloxane, octamethyltrisiloxane and cyclomethicone);
1 - 5 wt.% of a surfactant selected from polyoxyethylated fatty acid (10-18C)esters of sorbitan (e.g. Span 20, Span 80); (8-18C) fatty acid esters, and polyoxypropylene / polyoxyethylene block co-polymers (Poloxamers®);
1 - 8 wt.% of crospovidone, sodium croscarmellose and/or trihydroxystearin; and
1 - 3 wt.% of a divalent calcium and/or magnesium salt.

In another instance, the pharmaceutical composition comprises:
1 - 3 wt.% of daptomycin or a pharmaceutically acceptable salt thereof;
10 - 20 wt.% (6-18C) fatty acid esters (e.g. isopropyl myristate);
10 - 20 wt.% mono-, di- and/or triglycerides comprising glycerol and (6-18C) fatty acids;
50 - 70 wt.% silicone oils (such as, for example dimethicone, hexamethyldisiloxane, octamethyltrisiloxane and cyclomethicone);
1 - 5 wt.% of a surfactant selected from polyoxyethylated fatty acid (10-16C)esters of sorbitan (e.g. Span 20, Span 80); (8-16C) fatty acid esters, and polyoxypropylene / polyoxyethylene block co-polymers (Poloxamers®);
1 - 8 wt.% of crospovidone, sodium croscarmellose and/or trihydroxystearin; and
1 - 3 wt.% of a divalent calcium and/or magnesium salt.

In another instance, the pharmaceutical composition comprises:
1 - 3 wt.% of daptomycin or a pharmaceutically acceptable salt thereof;
10 - 40 wt.% isopropyl myristate;
10 - 60 wt.% caprylic/capric triglyceride/medium chain triglyceride;
0-30 wt.% dimethicone;
0 - 40 wt.% hexamethyldisiloxane;
0 - 30 wt.% cyclomethicone;
1 - 5 wt.% of a surfactant selected from polyoxyethylated fatty acid (10-16C)esters of sorbitan (e.g. Span 20, Span 80); (8-16C) fatty acid esters, and polyoxypropylene / polyoxyethylene block co-polymers (Poloxamers®);
1 - 8 wt.% of crospovidone, sodium croscarmellose and/or trihydroxystearin; and
1 - 3 wt.% of a divalent calcium and/or magnesium salt.

In another instance, the pharmaceutical composition comprises:
1 - 3 wt.% of daptomycin or a pharmaceutically acceptable salt thereof;
10 - 20 wt.% isopropyl myristate;
10 - 20 wt.% caprylic/capric triglyceride/medium chain triglyceride;
10 - 20 wt.% dimethicone;
10 - 40 wt.% hexamethyldisiloxane;
10 - 30 wt.% cyclomethicone;
1 - 5 wt.% of a surfactant selected from polyoxyethylated fatty acid (10-16C)esters of sorbitan (e.g. Span 20, Span 80); (8-16C) fatty acid esters, and polyoxypropylene / polyoxyethylene block co-polymers (Poloxamers®);
1 - 8 wt.% of crospovidone, sodium croscarmellose and/or trihydroxystearin; and
1 - 3 wt.% of a divalent calcium and/or magnesium salt.

### Preparation of the pharmaceutical compositions

The pharmaceutical compositions of the present disclosure can be prepared using conventional techniques known in the art.

The pharmaceutical compositions are suitably prepared by mixing (e.g. homogenising) all of the components together. If any volatile components are present as part of the oily vehicle (e.g. hexamethyldisiloxane or cyclomethicone), then it may be desirable to add these components following the initial mixing of the non-volatile components.

The individual components may be mixed (e.g. homogenised) by simply adding all of the components (optionally excluding any volatile components) at the same time into a mixing vessel and then mixing them all together (a "one-pot" mixture). Alternatively, the components may be added sequentially in two or more steps or stages.

Other experimental conditions requited to prepare the compositions, such as homogenisation times, homogenisation speeds, temperature control etc., can be readily determined by a person of ordinary skill in the art.

Further experimental details will also be evident from the accompanying Examples.

Once formed, the pharmaceutical compositions of the present disclosure may be loaded into a suitable delivery device, which may a metered dosage spray device or a device configured to dispense one or more droplets of the composition when actuated. Suitably, the device is a spray device.

### Delivery devices

The pharmaceutical compositions of the present disclosure may be loaded into a suitable delivery device for topical administration.

The selection of the delivery device will depend on the ultimate therapeutic application and the nature of the delivery required. In most cases, it is envisaged that the pharmaceutical compositions of the present disclosure will be administered as spot on or spray on formulations. As such, devices configured to deliver one or more defined volumes of the composition in the form of droplets, sprays or bolus of the composition of the pharmaceutical compositions are generally preferred.

Thus, in an instance, described herein is a device comprising a pharmaceutical composition as defined hereinbefore, wherein the device is configured to dispense a defined volume of the composition (for example, as a spray, jet, one or more droplets or bolus of the composition)..

The ability of the pharmaceutical compositions to spontaneously spread across the surface of the skin or mucosal surface to which it is applied means that a suitable spray of the pharmaceutical composition can provide a substantially uniform distribution of the antimicrobial agent on the skin surface.

### Therapeutic uses

The pharmaceutical compositions of the present disclosure are particularly suited to the treatment of topical infections. Once administered, the composition will spread across the surface of the skin and/or mucosal surface as previously described and the antimicrobial agent will then be released from the oily vehicle and dissolve in moisture present on the surface of the skin/mucosa.

The diseases that can be treated with the pharmaceutical compositions of the present disclosure will depend on the antimicrobial agent(s) selected for inclusion in the composition.

Examples of topical infections include impetigo, MRSA and CSSSI's (such as, for example, gangrene).

In instances where the antimicrobial agent is, or comprises, daptomycin, the pharmaceutical composition may be used for the treatment of infections caused by Gram-positive bacteria, especially *Staphylococcus aureus* bacteraemia (e.g. MRSA). In addition to its use for the treatment of MRSA, daptomycin is also used for the treatment of methicillin-susceptible *Staphylococcus aureus* (MSSA); streptococcal infections such as *Streptococcus pyrogenes*, *Streptococcus agalactiae*, *Streptococcus dysgalactiae*; and *Enterococcus faecalis.*

Daptomycin-containing compositions of the disclosure may also be used for the treatment of complicated skin and skin structure infections (cSSSIs) caused by complicated wound infections, such gangrene ("wet" and "dry" gangrene). Dry gangrene begins at the distal part of the limb due to ischemia, and often occurs in the toes and feet of elderly patients due to arteriosclerosis and thus, is also known as senile gangrene. Wet gangrene occurs in naturally moist tissue and organs such as the mouth, bowel, lungs, cervix, and bedsores occurring on body parts such as the sacrum, buttocks, and heels. Wet gangrene is characterised by numerous bacteria and has a poor prognosis (compared to dry gangrene) due to septicemia. In wet gangrene, the tissue is infected by saprogenic microorganisms (*Clostridium perfringens* or *Bacillus fusiformis*, for example), which cause tissue to swell and emit a fetid smell.

Daptomycin-containing compositions could also be used alone or in combination with other antibiotics, e.g. polymixin B, to yield a broad spectrum of activity.

A particular application of pharmaceutical compositions of the disclosure comprising daptomycin is the treatment of MRSA infection. MRSA infection is major issue in hospitals and there is a pressing need for pharmaceutical compositions that can be used topically to treat MRSA present on the skin or mucosal surfaces. There is a particular need for topical pharmaceutical compositions that can be used to eradicate MRSA from the skin or mucosal surfaces prior to elective surgical procedures.

Pharmaceutical compositions of the disclosure comprising other antibacterial agents may be used for the treatment of any topical infections that are sensitive to antimicrobial agent concerned. For example, pharmaceutical compositions comprising vancomycin may also be used for the treatment of topical infection caused by Gram-positive bacteria. Pharmaceutical compositions comprising mupirocin may also be used for the treatment of topical infection caused by Gram-positive bacteria such as MRSA and MSSA.

The dosage required will vary depending on the antimicrobial agent and its intended use. The actual dosage levels of the antimicrobial agents in the pharmaceutical compositions may be varied so as to obtain an amount of the active compound(s) that is effective to achieve the desired therapeutic response for a particular patient, compositions, and mode of administration. The selected dosage level will depend upon the activity of the particular compound, the method of administration, the severity of the condition being treated and the condition and prior medical history of the patient being treated. However, it is within the skill of the art to start doses of the compound at levels lower than required for to achieve the desired therapeutic effect and to gradually increase the dosage until the desired effect is achieved. The pharmaceutical compositions of the disclosure may also be administered on a regimen of 1 to 4 times per day, e.g. once or twice per day. The dosage regimen may be adjusted to provide the optimal therapeutic response.

Suitably, the appropriate dosage can be easily administered by means of dosage device as previously described.

The pharmaceutical compositions of the present disclosure may be used on their own as the sole therapy. Alternatively, the compositions may be administered as part of a combination therapy with one or more other antimicrobial treatments. Such conjoint treatment may be achieved by way of the simultaneous, sequential or separate administration of the individual components of the treatment.

### Examples

### Materials

The following materials were used in the preparation of the formulations described herein:

**Table 1 - Materials**

| **Component** | **Chemical/common name** | **Supplier** |
|---|---|---|
| Daptomycin | Daptomycin | |
| Labrafac PG | Propylene glycol dicaprylate/dicaprate | Gattefosse |
| IPM | Isopropylmyristate, Isopropyl tetradecanoate | Mistral Chemicals |
| Labrafac lipophile | Labrafac Lipophile WL 1349, Medium chain triglycerides | Gattefosse |
| Capryol 90 | Propylene glycol monocaprylate | Gattefosse |
| Capmul GMO-50 | Glycerol monooleate | Abitec |
| Captex 355 | Triglycerides of caprylic/capric acid | Abitec |
| Ca stearate | Calcium stearate, stearic acid calcium salt | Sigma-Aldrich |
| Kollidon CL-M | Crosslinked polyvinylpyrrolidone, micronized (Crospovidone) | BASF |
| Span 20 | Sorbitan monolaurate | Croda |
| Span 80 | Sorbitan monooleate | Croda |
| Tween 80 | Polyethylene glycol sorbitan monooleate | Croda |
| Thixcin R | 1,2,3-Propanetriyl tris(12-hydroxyoctadecanoate | Elementis |
| Vaseline | Petrolatum, meets specification of Ph. Eur. | Sigma-Aldrich |
| Silicone fluid 20cSt | Dimethicone NF | Dow Corning |
| ST silicone 0.65cSt | Hexamethyldisiloxane | Dow Corning |
| ST silicone 1cSt | Octamethyltrisiloxane | Dow Corning |
| Cyclomethicone 5 NF | Decamethylcyclopentasiloxane | Dow Corning |

Daptomycin is sourced commercially or wet-milled to give particles within the desired size range.

### General methods

### Formulation preparation

### Method A. Preparation of anhydrous topical suspension formulations for physical inspection

A one-pot homogenisation preparation method was developed for prototype suspension formulations. Any volatile components/excipients (e.g. hexamethyldisiloxane (0.65cSt), octamethyltrisiloxane or cyclomethicone) were incorporated into the formulation following homogenisation.

Formulations were prepared on either a 10g or 15g scale in 20ml glass vial (Chromacol, UK) on a w/w basis (using either 2-place or 5-place laboratory balances). All components, excluding any volatile excipients, were transferred (solids first) to a single vial. Vial contents were homogenised for a total of 4 minutes (PowerGen 700 with a 7mm probe on setting 4, -10,000 rpm, 2 x 2 minutes with vial cooling between sessions). Any volatile components were added (by weight) directly to the formulation vial. Vials were immediately tightly capped and shaken.

### Method B. Prototype anhydrous topical suspension formulations containing Thixcin R for physical inspection (Example 5; Formulations S to U described herein)

To avoid unnecessary heating of daptomycin during the preparation of formulations containing Thixcin R the following approach below was used. Formulations were prepared on a 10g scale in 20ml glass vial (Chromacol, UK) on a w/w basis (using either 2-place or 5-place laboratory balances).

Daptomycin was wet milled to produce a fine dispersion by mixing under high shear for between 30 minutes and 1 hour to form a daptomycin concentrate.

Formulation components, excluding the dispersed daptomycin and associated Labrafac PG, were transferred to a vial and heated to 90-95°C with stirring (magnetic follower). Earlier investigations indicated that elevated temperature appeared to provide improved results compared to Thixcin R preparation at ∼45-50°C. For formulation S described herein, the mixture was homogenised for a total of 4-5 minutes. For formulations T and U described herein homogenisation at elevated temperature was not used (to reduce foaming) and the mixtures were instead stirred for ≥30 minutes. The Thixcin R formulation mixtures were then allowed to cool under stirring to ∼50°C. At an appropriate time the wet milled daptomycin and associated Labrafac PG was warmed to ∼50°C with stirring and 1.344ml added via Microman positive displacement pipette to the Thixcin R mixture and the formulation mixed (magnetic follower) at that temperature for a short period until homogeneous. The selected volume was chosen to correspond to an addition of 1.4g daptomycin/Labrafac PG but subsequent calculations indicated that this was equivalent to ∼1.29g (and thus daptomycin loading was ∼1.87%). Formulation S was allowed to cool to room temperature under stirring. Formulation T was homogenized for a total of 4 minutes at ∼50°C then allowed to cool under stirring. Formulation U was homogenized for a total of 2 minutes at ∼50°C, allowed to cool under stirring, then homogenized for a further 4 minutes at room temperature.

### Method C. Preparation of anhydrous topical suspension formulations for stability assessment (Example 1; Formulations A to D and F to H described herein)

Formulations were prepared as follows. All non-volatile components excluding daptomycin and a proportion of the IPM (present in the wet milled API) were transferred to a 20ml glass vial. The vial containing wet milled API was vigorously stirred (magnetic follower) and appropriate quantities were transferred (via a positive displacement pipette and Pasteur pipette) to each formulation vial (via weight). Each suspension formulation (Formulation A-D, F-H) was then homogenised for four minutes with vial cooling. Any volatile components (Formulations F-H) were then added, and the suspensions briefly homogenised (10 seconds), capped and shaken.

### Example 1 - Preparation of formulations A to D and F to H

The formulations outlined in Tables 2 and 3 below were each prepared on a 15g scale using Method C outlined above. Fresh quantities of daptomycin were transferred from the bulk container and water content was measured (Aquamax KF Coulometric) in triplicate and the absolute purity of the daptomycin calculated (92.7% and 92.3% for Tables 2 and 3, respectively). The daptomycin content of each formulation was adjusted to a target 2% using the calculated absolute purity.

An example of the component weights (for Formulations F-H) is shown in Table 4.

**Table 2 - Non-aqueous suspension formulations for informal stability assessment (15g batches, Formulations A-D suspensions, Formulation E ointment)**

| Component | Formulation % (w/w) | Formulation B % (w/w) | Formulation % (w/w) | Formulation D % (w/w) |
|---|---|---|---|---|
| Daptomycin* | 2(2.16) | 2(2.16) | 2(2.16) | 2(2.16) |
| Labrafac PG | 12 | 12 | - | - |
| IPM | 26 | 27 | 35 | 36 |
| Labrafac lipophile | 45.84 | 46.84 | 53.84 | 54.84 |
| Capryol 90 | 5 | 5 | - | - |
| Ca stearate | 2 | - | 2 | - |
| Kollidon CL-M | 5 | 5 | 5 | 5 |
| Span 20 | 2 | 2 | 2 | 2 |
| Vaseline | - | - | - | - |

| | | | | |
|---|---|---|---|---|
| * API content corrected for absolute purity (chemical purity and measured water content) | | | | |

**Table 3 - Non-aqueous suspension formulations for informal stability assessment (15g batches, Formulations F-H suspensions)**

| Component | Formulation F % (w/w) | Formulation G % (w/w) | Formulation H % (w/w) |
|---|---|---|---|
| Daptomycin* | 2(2.17) | 2(2.17) | 2(2.17) |
| IPM | 15 | 15 | 15 |
| Labrafac lipophile | 13.83 | 13.83 | 13.83 |
| Ca stearate | 2 | 2 | 2 |
| Kollidon CL-M | 5 | 5 | 5 |
| Span 20 | 2 | 2 | 2 |
| Silicone fluid 20cSt | 15 | 15 | - |
| ST silicone 0.65cSt** | 30 | 45 | 35 |
| Cyclomethicone 5 NF** | 15 | - | 25 |

| | | | |
|---|---|---|---|
| * API content corrected for absolute purity (chemical purity and measured water content) ** Volatile components | | | |

**Table 4 - Construction procedure for prototype non-aq suspension formulations F-H containing 2% daptomycin (15g preparation scale)**

| Component/phase | Formulation F | Formulation G | Formulation H |
|---|---|---|---|
| **Phase A (to be added to B)** | Weight (g) | Weight (g) | Weight (g) |
| Wet milled API in IPM (16% w/w) | 2.032 | 2.032 | 2.032 |
| *IPM component* | *1.707* | *1.707* | 1.707 |
| *Daptomycin component* | *0.325** *(0.300)* | *0.325** *(0.300)* | *0.325** *(0.300)* |
| | | | |

| **Phase B** | | | |
|---|---|---|---|
| Kollidon CL-M | 0.750 | 0.750 | 0.750 |
| Ca stearate | 0.300 | 0.300 | 0.300 |
| Span 20 | 0.300 | 0.300 | 0.300 |
| IPM | 0.543 | 0.543 | 0.543 |
| Labrafac lipophile | 2.075 | 2.075 | 2.075 |
| Silicone fluid 20cSt | 2.250 | 2.250 | 0.000 |
| | | | |

| **Phase C (added post homogenisation)** | | | |
|---|---|---|---|
| ST silicone 0.65cSt** | 4.500 | 6.750 | 5.250 |
| Cyclomethicone 5 NF** | 2.250 | 0.000 | 3.750 |
| | | | |
| Total (g) | 15.000 | 15.000 | 15.000 |

| | | | |
|---|---|---|---|
| * API content corrected for absolute purity (chemical purity and measured water content) ** Volatile components | | | |

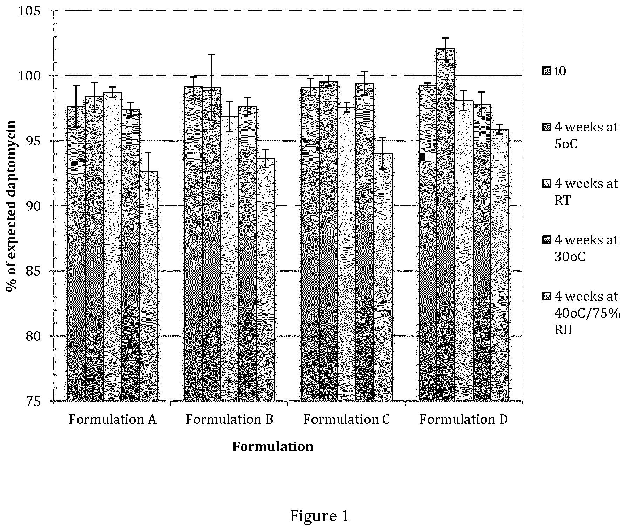

The percentage recovery of daptomycin from Formualtions A-D over time is shown in Figure 1.

### Example 2 - Preparation of formulations I to L

Using the procedure described in Method A above, the following formulations I to L were prepared:

**Table 5 - Non-aqueous suspension formulations I to L**

| Component | Formulation I % (w/w) | Formulation J % (w/w) | Formulation K % (w/w) | Formulation L % (w/w) |
|---|---|---|---|---|
| Daptomycin | 2 | 2 | 2 | 2 |
| Labrafac PG | 12 | 12 | 12 | 12 |
| IPM | 34 | 34 | 28 | 32 |
| Captex 355 | 48 | 48 | 48 | 48 |
| Ca stearate | - | 1 | 2 | 2 |
| Capmul GMO | - | - | 5 | - |
| Kollidon CL-M | - | 1 | 1 | 2 |
| Span 20 | - | - | - | 2 |
| Span 80 | 3 | 2 | 2 | - |
| Tween 80 | 1 | - | - | - |

### Example 3 - Preparation of formulations M to O

Using the procedure described in Method A above, the following formulations M to O were prepared:

**Table 6 - Non-aqueous suspension formulations M to O**

| Component | Formulation M % (w/w) | Formulation N % (w/w) | Formulation O % (w/w) |
|---|---|---|---|
| Daptomycin | 2 | 2 | 2 |
| Labrafac PG | 12 | 12 | 12 |
| IPM | 27 | 29 | 29 |
| Labrafac lipophile | 48 | 48 | 48 |
| Capryol 90 | 5 | 5 | 5 |
| Ca stearate | 2 | 2 | - |
| Kollidon CL-M | 2 | - | 2 |
| Span 20 | 2 | 2 | 2 |

### Example 4 - Preparation of formulations P to R

Using the procedure described in Method A above, the following formulations P to R were prepared:

**Table 7 - Non-aqueous suspension formulations P to R**

| Component | Formulation P % (w/w) | Formulation Q % (w/w) | Formulation R % (w/w) |
|---|---|---|---|
| Daptomycin | 2 | 2 | 2 |
| Labrafac PG | 12 | - | - |
| IPM | 27 | 35 | 35 |
| Labrafac lipophile | 47 | 57 | 54 |
| Capryol 90 | 5 | - | - |
| Ca stearate | - | 2 | 2 |
| Kollidon CL-M | 5 | 2 | 5 |
| Span 20 | 2 | 2 | 2 |

### Example 5 - Preparation of formulations S to U

Using the procedure described in Method B above, the following formulations S to U were prepared:

**Table 8 - Non-aqueous suspension formulations S to U**

| Component | Formulation S % (w/w) | Formulation T % (w/w) | Formulation U % (w/w) |
|---|---|---|---|
| Daptomycin* | 2 | 2 | 2 |
| Labrafac PG | 12 | 12 | 12 |
| IPM | 29.4 | 29.7 | 25.8 |
| Labrafac lipophile | 47 | 47 | 46 |
| Capryol 90 | 5 | 5 | 5 |
| Ca stearate | 2 | 2 | 2 |
| Thixcin R | 0.6 | 0.3 | 0.2 |
| Span 20 | 2 | 2 | 2 |
| Kollidon CL-M | - | - | 5 |

| | | | |
|---|---|---|---|
| * daptomycin milled at 14.3% in Labrafac PG (060213) | | | |

### Example 6 - Preparation of formulations V and W

Using the procedure described in Method A above, the following formulations V and W were prepared:

**Table 9 - Non-aqueous suspension formulations V and W**

| Component | Formulation V % (w/w) | Formulation W % (w/w) |
|---|---|---|
| Daptomycin | 2 | 2 |
| IPM | 24 | 19 |
| Labrafac lipophile | 15 | - |
| Ca stearate | 2 | 2 |
| Kollidon CL-M | 5 | 5 |
| Span 20 | 2 | 2 |
| Silicone fluid 20cSt | 20 | 20 |
| ST silicone 0.65cSt | 20 | 30 |
| ST silicone 1cSt | 10 | 20 |

### Example 7 - Preparation of formulation X

Using the procedure described in Method A above, the following formulation X was prepared:

**Table 10 - Non-aqueous suspension formulation X**

| Component | Formulation X % (w/w) |
|---|---|
| Daptomycin | 2 |
| IPM | 15 |
| Labrafac lipophile | 14 |
| Ca stearate | 2 |
| Kollidon CL-M | 5 |
| Span 20 | 2 |
| Silicone fluid 20cSt | 15 |
| ST silicone 0.65cSt | 45 |

### Example 8 - Preparation of formulation Y

Using the procedure described in Method A above, the following formulation Y was prepared:

**Table 11 - Non-aqueous suspension formulation Y**

| Component | Formulation Y % (w/w) |
|---|---|
| Daptomycin | 2 |
| IPM | 15 |
| Labrafac lipophile | 14 |
| Ca stearate | 2 |
| Kollidon CL-M | 5 |
| Span 20 | 2 |
| Silicone fluid 20cSt | 10 |
| ST silicone 0.65cSt | 40 |
| Cyclomethicone 5 NF | 10 |

### Example 9 - Preparation and assessment of formulations Z and AA

Using the procedure described in Method A above, the following formulations Z and AA were prepared:

**Table 12 - Non-aqueous suspension formulations Z and AA**

| Component | Formulation Z % (w/w) | Formulation AA % (w/w) |
|---|---|---|
| Daptomycin | 2 | 2 |
| IPM | 15 | 15 |
| Labrafac lipophile | 14 | 14 |
| Ca stearate | 2 | 2 |
| Kollidon CL-M | 5 | 5 |
| Span 20 | 2 | 2 |
| Silicone fluid 20cSt | 15 | 0 |
| ST silicone 0.65cSt | 30 | 30 |
| Cyclomethicone 5 NF | 15 | 30 |

### Example 10 - Preparation of formulations BB and CC

Using the procedure described in Method A above, the following formulations BB and CC were prepared:

**Table 13 - Non-aqueous suspension formulations BB and CC**

| Component | Formulation BB % (w/w) | Formulation CC % (w/w) |
|---|---|---|
| Daptomycin | 2 | 2 |
| IPM | 15 | 15 |
| Labrafac lipophile | 14 | 14 |
| Ca stearate | 2 | 2 |
| Kollidon CL-M | 5 | 5 |
| Span 20 | 2 | 2 |
| Silicone fluid 20cSt | 5 | 0 |
| ST silicone 0.65cSt | 45 | 35 |
| ST silicone 1cSt | 0 | 0 |
| Cyclomethicone 5 NF | 10 | 25 |

### Example 11 - Assessment of sedimentation volumes

Formulations were prepared for physical assessment on 10g or 15g scales as described aboce in reference to Examples 1 to 10 and were visually assessed over various time-periods for sedimentation and re-suspendability of solids.

Formulations were stored in 20ml glass vials and the height of the solids/liquids monitored. Sedimentation volume, F, was estimated using the liquid (V₀) and solid heights (V_{H}) measured using vernier calipers, with the vial base height subtracted from each measurement prior to calculation. For example, Formulation F described herein at 5 days, F = V_{H}/V₀ = 13.9mm/31.8mm = ∼44%, and upon shaking (hand-shaken 3 times) solids were easily re-suspended. Further assessment at 10 days and 24 days produced F values of ∼40% and ∼48% respectively, and solids continued to be easily re-suspended.

Approximate sedimentation volumes for the Formulations A to CC at various time points are shown in Table 14 below.

**Table 14 - approximate sedimentation values**

| Formulation | Days since preparation | Approx. sedimentation volume |
|---|---|---|
| A | 28 | 57% |
| | 96 | 68% |
| B | 28 | 51% |
| | 96 | 58% |
| C | 28 | 67% |
| | 96 | 61% |
| D | 28 | 62% |
| | 96 | 63% |
| F | 7 | 58% |
| G | 7 | 55% |
| H | 7 | 55% |
| I | 1 | 33% |
| J | 1 | 22% |
| | 154 | 18% |
| K | 1 | 30% |
| | 154 | 27% |
| L | 1 | 52% |
| | 2 | 50% |
| | 154 | 40% |
| M | 1 | 46% |
| | 4 | 45% |
| | 7 | 41% |
| | 12 | 42% |
| | 27 | 43% |
| | 153 | 38% |
| N | 1 | 44% |
| | 4 | 38% |
| | 27 | 29% |
| | 153 | 23% |
| O | 1 | 51% |
| | 4 | 43% |
| | 27 | 39% |
| | 153 | 40% |
| P | 3 | 65% |
| | 6 | 59% |
| | 11 | 60% |
| | 26 | 57% |
| | 155 | 53% |
| Q | 3 | 51% |
| | 6 | 39% |
| | 11 | 47% |
| | 26 | 41% |
| | 155 | 35% |
| R | 2 | 71% |
| | 7 | 65% |
| | 22 | 66% |
| | 151 | 46% |
| S | 124 | ∼83% |
| T | 1 | very small amount of sedimentation |
| | 9 | very small amount of sedimentation |
| | 113 | not separated |
| U | 3 | 69% |
| | 105 | 70% |
| V | 1 | 56% |
| | 4 | 54% |
| | 11 | 52% |
| | 25 | 54% |
| W | 1 | 47% |
| | 4 | 40% |
| W | 11 | 38% |
| | 25 | 44% |
| X | 3 | 44% |
| | 10 | 40% |
| | 24 | 48% |
| Y | 31 | 44% |
| Z | 32 | 43% |
| AA | 1 | 48% |
| | 6 | 48% |
| | 17 | 46% |
| BB | 3 | 47% |
| | 14 | 42% |
| CC | 3 | 48% |
| | 14 | 41% |

All formulations could be re-suspended.

### Example 12 - Spreading assessments

Selected formulations (Formulations A-D and F-G) were assessed for spreadability on glass surfaces and human skin.

Glass petri dishes (90mm diameter) were located above a ruler and 100 µl of formulation applied in the centre of the petri dish. The spreading (extent and speed) for the assessed formulations was recorded and assessed. By comparison, water (100 µl) applied in an identical manner produced a discrete drop of ∼11mm diameter which remained static between 10 seconds and 25 minutes. For formulations A-D described herein, the formulation spread rapidly and both the solid and liquid components reached a diameter of ∼18-21mm over a period of ≤15seconds and the diameter remained in that range for a further 25 minutes. For formulations F-G described herein, the formulation spread rapidly and solid components reached a diameter of ∼38-43mm and the liquid components a diameter of ∼43-52mm over a period of -34-37 seconds, then appeared static.

Application to human skin was assessed by transferring a (10 µl) drop to human skin (horizontal) and observing the self-spreading of the formulation over 2 minutes. By comparison, water (10 µl) applied in an identical manner produced a discrete drop of ∼3mm diameter (∼0.07cm²) which remained static for the 2 minute observation period. Formulations A-D spread rapidly without further intervention from the applicator and by 10 seconds each formulation had covered ≥0.3cm² and this increased further to ≥0.5cm² over the 2 minutes, at which point the rate of spreading had slowed greatly. Formulations F-G spread rapidly without further intervention from the applicator and by 10 seconds each formulation had covered ≥1cm² and this increased further over the 2 minutes, ranging from ∼2 to 5 cm², at which point the rate of spreading had slowed greatly due to the considerable loss of volatile components.

## Claims

1. A pharmaceutical composition suitable for topical application to the body, said composition comprising daptomycin or a pharmaceutically acceptable salt thereof that is dissolved and/or suspended within an oily vehicle, wherein the daptomycin or pharmaceutically acceptable salt thereof is present in an amount of 0.01 wt.% to 10 wt.% of the total composition, and wherein the moisture content of the composition is less than 1 wt.%.

2. A pharmaceutical composition according to claim 1, wherein:
(a) the daptomycin or pharmaceutically acceptable salt thereof is uniformly dispersed or dispersible within the oily vehicle; and/or
(b) the daptomycin or pharmaceutically acceptable salt thereof is present as discrete particles, optionally wherein the particles have a D90 of 75µm or less.

3. A pharmaceutical composition according to any one of the preceding claims, wherein the oily vehicle:
(a) comprises one or more oil components selected from the group consisting of:
mono-, di- and/or triglycerides comprising glycerol and (6-18C) fatty acids;
propylene glycol esters of (6-18C) fatty acids;
(6-18C) fatty acid esters;
(6-18C) fatty alcohols;
silicone fluids/oils; and
lanolin/lanolin derivatives; and/or
(b) constitutes between 70 wt.% and 99.5 wt.% of the composition; and/or
(c) comprises an oily emollient and said emollient constitutes up to 70% wt.% of the composition; and/or
(d) comprises one or more oil components selected from the group consisting of:
mono-, di- and/or tri-glycerides comprising glycerol and (6-18C) fatty acids;
propylene glycol esters of (6-18C) fatty acids;
(6-18C) fatty acid esters (e.g. isopropyl myristate); and
silicone fluids/oils; and/or
(e) comprises:
5 - 45 wt.% (6-18C) fatty acid esters;
5 - 60 wt.% mono-, di- and/or tri-glycerides comprising glycerol and (6-18C) fatty acids;
0 - 10 wt.% propylene glycol esters of (6-18C) fatty acids;
0 - 70 wt.% silicone fluids/oils; and
13 - 5 wt.% lanolin/lanolin derivative.

4. A pharmaceutical composition according to any one of the preceding claims, wherein the composition oily vehicle further comprises a suspending agent.

5. A pharmaceutical composition according to claim 4, wherein the suspending agent:
(a) is selected from one or more of the following:
cross-linked polymers suitably in a micronized form, e.g PVP (crospovidone) & sodium croscarmellose;
clays: micronized clays aluminium phyllosilicate (bentonite);
magnesium/aluminium silicates;
silicas: silica (e.g. Syliod®, Cab-o-sil®), silicon dioxide (e.g. Aerosil®); and
hydrogenated oils: e.g. trihydroxystearin (Thixcin® R); and/or
(b) is crospovidone; and/or
(c) is present in an amount of 0.2 - 15 wt.% of the composition.

6. A pharmaceutical composition according to any one of the preceding claims, wherein the oily vehicle further comprises one or more oily surfactants.

7. A pharmaceutical composition according to claim 6, wherein the oily surfactant:
(a) is present in an amount of up to 7.5 wt.% of the total composition; and/or
(b) has a HLB (hydrophilic-lipophilic balance) value of less than 7; and/or
(c) is selected from the group consisting of: polyoxyethylated fatty acid (10-18C)esters of sorbitan (e.g. Span 20, Span 80); (8-18C) fatty acid esters, and polyoxypropylene / polyoxyethylene block co-polymers (Poloxamers®).

8. A pharmaceutical composition according to any one of the preceding claims, wherein the pharmaceutical composition comprises a suspending agent according to claim 4 or 5 and an oily surfactant according to claim 6 or 7.

9. A pharmaceutical composition according to any one of the preceding claims, the composition further comprises a divalent metal salt stabiliser, optionally selected from calcium and/or magnesium fatty acid salts (e.g. calcium and/or magnesium stearate), calcium and/or magnesium sulphate, calcium and/or magnesium trisilicate, and calcium and/or magnesium carbonate, optionally wherein the amount of stabiliser present is 0 - 5 wt.% of the total composition.

10. A pharmaceutical composition according to any one of the preceding claims, wherein the composition comprises:
(i) 0.05 - 5 wt.% of daptomycin or a pharmaceutically acceptable salt thereof;
(ii) 75 - 99.5 wt.% of an oily vehicle; and
(iii) optionally 0.5 - 10 wt.% of a suspending agent.

11. A pharmaceutical composition according to any one of the preceding claims, wherein the composition comprises:
0.05 - 5 wt.% of daptomycin or a pharmaceutically acceptable salt thereof;
5 - 45 wt.% (6-18C) fatty acid esters (e.g. isopropyl myristate);
5 - 60 wt.% mono-, di- and/or triglycerides comprising glycerol and (6-18C) fatty acids;
0 - 10 wt.% propylene glycol esters of (6-18C) fatty acids;
0 - 70 wt.% silicone oils (such as, for example dimethicone, hexamethyldisiloxane, octamethyltrisiloxane and cyclomethicone);
0 - 5 wt.% lanolin/lanolin derivative;
up to 7.5 wt.% of a surfactant selected from polyoxyethylated fatty acid (10-18C)esters of sorbitan (e.g. Span 20, Span 80); (8-18C) fatty acid esters, and polyoxypropylene / polyoxyethylene block co-polymers (Poloxamers®);
0.5 - 10 wt.% of a suspending agent selected from one or more of the following:
cross-linked polymers suitably in a micronized for, e.g PVP (crospovidone) & sodium croscarmellose;
clays: micronized clays aluminium phyllosilicate (bentonite);
magnesium/aluminium silicates;
silicas: silica (Syliod®, Cab-o-sil®), silicon dioxide (Aerosil®); and
hydrogenated oils: trihydroxystearin (Thixcin® R); and
0 - 5 wt.% of a divalent metal salt.

12. A pharmaceutical composition according to any one of the preceding claims, wherein the composition comprises:
0.2 - 4 wt.% of daptomycin or a pharmaceutically acceptable salt thereof;
10 - 40 wt.% isopropyl myristate;
10 - 60 wt.% caprylic/capric triglyceride/medium chain triglyceride;
0 - 30 wt.% dimethicone;
0 - 40 wt.% hexamethyldisiloxane;
0 - 30 wt.% cyclomethicone;
1 - 5 wt.% of a surfactant selected from polyoxyethylated fatty acid (10-16C)esters of sorbitan (e.g. Span 20, Span 80); (8-16C) fatty acid esters, and polyoxypropylene / polyoxyethylene block co-polymers (Poloxamers®);
1 - 8 wt.% of crospovidone, sodium croscarmellose and/or trihydroxystearin; and
1 - 3 wt.% of a divalent calcium and/or magnesium salt.

13. A pharmaceutical composition according to any one of claims 1 to 12 for use in the treatment of topical infections, optionally wherein said topical infection is selected from impetigo, MRSA or a complicated skin and/or skin structure infection (cSSSI).

14. A pharmaceutical composition according to any one of claims 1 to 12 for use in the eradication of topical MRSA prior to surgery.

15. A method of preparing a pharmaceutical composition according to claim 1, said method comprising suspending daptomycin or a pharmaceutically acceptable salt thereof in an oily vehicle.

16. A device comprising a pharmaceutical composition according to any one of claims 1 to 12, wherein the device is configured to dispense a volume of the composition for topical application.

## Patentansprüche

1. Pharmazeutische Zusammensetzung, die für die topische Anwendung auf dem Körper geeignet ist, wobei die Zusammensetzung Daptomycin oder ein pharmazeutisch unbedenkliches Salz davon umfasst, das innerhalb eines Ölvehikels aufgelöst und/oder suspendiert ist, wobei das Daptomycin oder pharmazeutisch unbedenkliche Salz davon in einer Menge von 0,01 Gew.-% bis 10 Gew.-% der gesamten Zusammensetzung vorhanden ist, und wobei der Feuchtigkeitsgehalt der Zusammensetzung weniger ist als 1 Gew.-%.

2. Pharmazeutische Zusammensetzung nach Anspruch 1, wobei:
(a) das Daptomycin oder pharmazeutisch unbedenkliches Salz davon gleichmäßig innerhalb des Ölvehikels dispergiert oder dispergierbar ist; und/oder
(b) das Daptomycin oder ein pharmazeutisch unbedenkliches Salz davon als diskrete Partikel vorhanden ist, wahlweise wobei die Partikel eine D90 von 75 µm oder weniger aufweisen.

3. Pharmazeutische Zusammensetzung nach einem der vorhergehenden Ansprüche, wobei das Ölvehikel:
(a) eine oder mehrere Ölkomponenten umfasst, die ausgewählt sind aus der Gruppe bestehend aus:
Mono-, Di- und/oder Triglyceriden, die Glycerin und (6-18C)Fettsäuren umfassen;
Propylenglycolestern von (6-18C)Fettsäuren;
(6-18C)Fettsäureester;
(6-18C)Fettalkohole;
Siliconflüssigkeiten/-öle; und
Lanolin/Lanolinderivate; und/oder
(b) zwischen 70 Gew.-% und 99,5 Gew.-% der Zusammensetzung bildet; und/oder
(c) einen öligen Weichmacher umfasst und der Weichmacher bis zu 70 Gew.-% der Zusammensetzung bildet; und/oder
(d) eine oder mehrere Ölkomponenten umfasst, die ausgewählt sind aus der Gruppe bestehend aus:
Mono-, Di- und/oder Triglyceriden, die Glycerin und (6-18C)Fettsäuren umfassen;
Propylenglycolestern von (6-18C)Fettsäuren;
(6-18C)Fettsäureester (z. B. Isopropylmyristat); und
Siliconflüssigkeiten/-öle; und/oder
(e) Folgendes umfasst:
5-45 Gew.-% (6-18C)Fettsäureester;
5-60 Gew.-% Mono-, Di- und/oder Triglyceride, die Glycerin und (6-18C)Fettsäuren;
0-10 Gew.-% Propylenglycolestern von (6-18C)Fettsäuren;
0-70 Gew.-% Siliconflüssigkeiten/-öle; und
13-5 Gew.-% Lanolin/Lanolinderivat.

4. Pharmazeutische Zusammensetzung nach einem der vorhergehenden Ansprüche, wobei das Zusammensetzungsölvehikel ferner ein Suspensionsmittel umfasst.

5. Pharmazeutische Zusammensetzung nach Anspruch 4, wobei das Suspensionsmittel:
(a) ausgewählt ist aus einem oder mehreren der Folgenden:
vernetzte Polymere, geeignet in einer mikronisierten Form, z. B. PVP (Crospovidon) & Natriumcroscarmellose;
Ton: mikronisiertes Ton-Aluminium-Schichtsilikat (Bentonit);
Magnesium-/Aluminiumsilikate;
Kieselsäuren: Kieselsäure (z. B. Syliod®, Cab-o-sil®), Siliciumdioxid (z. B. Aerosil®);
und
hydrierte Öle: z. B. Trihydroxystearin (Thixcin® R); und/oder
(b) Crospovidon ist; und/oder
(c) in einer Menge von 0,2-15 Gew.-% der Zusammensetzung vorhanden ist.

6. Pharmazeutische Zusammensetzung nach einem der vorhergehenden Ansprüche, wobei das Ölvehikel ferner ein oder mehrere ölige Tenside umfasst.

7. Pharmazeutische Zusammensetzung nach Anspruch 6, wobei das ölige Tensid:
(a) in einer Menge von 7,5 Gew.-% der Gesamtzusammensetzung vorhanden ist; und/oder
(b) einen Hydrophil-Lipophil-Gleichgewichts- (hydrophilic-lipophilic balance - HLB) Wert von weniger als 7 aufweist; und/oder
(c) ausgewählt wird aus der Gruppe bestehend aus: polyoxyethylierte Fettsäure-(10-18C)Ester von Sorbitan (z. B. Span 20, Span 80); (8-18C)Fettsäureester und Polyoxypropylen / Polyoxyethylenblock-Copolymere (Poloxamers®).

8. Pharmazeutische Zusammensetzung nach einem der vorhergehenden Ansprüche, wobei die pharmazeutische Zusammensetzung ein Suspensionsmittel nach Anspruch 4 oder 5 umfasst und ein öliges Tensid nach Anspruch 6 oder 7.

9. Pharmazeutische Zusammensetzung nach einem der vorhergehenden Ansprüche, wobei die Zusammensetzung ferner einen zweiwertigen Metallsalzstabilisator umfasst, wahlweise ausgewählt aus Calcium- und/oder Magnesium-Fettsäuresalzen (z. B. Calcium und/oder Magnesiumstearat), Calcium- und/oder Magnesiumsulfat, Calcium- und/oder Magnesiumtrisilikat und Calcium- und/oder Magnesiumcarbonat, wahlweise, wobei die Menge von vorhandenem Stabilisator 0-5 Gew.-% der Gesamtzusammensetzung ist.

10. Pharmazeutische Zusammensetzung nach einem der vorhergehenden Ansprüche, wobei die Zusammensetzung Folgendes umfasst:
(i) 0,05-5 Gew.-% von Daptomycin oder ein pharmazeutisch unbedenkliches Salz davon;
(ii) 75-99,5 Gew.-% eines Ölvehikels; und
(iii) wahlweise 0,5-10 Gew.-% eines Suspensionsmittels.

11. Pharmazeutische Zusammensetzung nach einem der vorhergehenden Ansprüche, wobei die Zusammensetzung Folgendes umfasst:
0,05-5 Gew.% von Daptomycin oder ein pharmazeutisch unbedenkliches Salz davon;
5-45 Gew.-% (6-18C)Fettsäureester (z. B. Isopropylmyristat);
5-60 Gew.-% Mono-, Di- und/oder Triglyceride, die Glycerin und (6-18C)Fettsäuren;
0-10 Gew.-% Propylenglycolestern von (6-18C)Fettsäuren;
0-70 Gew.-% Siliconöle (wie etwa Dimethicon, Hexamethyldisiloxan, Octamethyltrisiloxan und Cyclometicon);
0-5 Gew.-% Lanolin/Lanolinderivat;
Bis zu 7,5 Gew.-% eines Tensids ausgewählt aus polyoxyethyliertem Fettsäure-(10-18C)Ester von Sorbitan (z. B. Span 20, Span 80); (8-18C)Fettsäureester und Polyoxypropylen / Polyoxyethylenblock-Copolymere (Poloxamers®);
0,5-10 Gew.-% eines Suspensionsmittels ausgewählt von einem oder mehreren der Folgenden:
vernetzte Polymere, geeignet in einer mikronisierten Form, z. B. PVP (Crospovidon) & Natriumcroscarmellose;
Ton: mikronisiertes Ton-Aluminium-Schichtsilikat (Bentonit);
Magnesium-/Aluminiumsilikate;
Kieselsäuren: Kieselsäure (Syliod®, Cab-o-sil®), Siliciumdioxid (Aerosil®); und
hydrierte Öle: Trihydroxystearin (Thixcin® R); und 0-5 Gew.-% eines zweiwertigen Metallsalzes.

12. Pharmazeutische Zusammensetzung nach einem der vorhergehenden Ansprüche, wobei die Zusammensetzung Folgendes umfasst:
0,2-4 Gew.% von Daptomycin oder ein pharmazeutisch unbedenkliches Salz davon;
10-40 Gew-% Isopropylmyristat;
10-60 Gew.-% Capryl-/Caprin-Triglycerid/mittelkettiges Triglycerid;
0-30 Gew.-% Dimethicon;
0-40 Gew.-% Hexamethyldisiloxan;
0-30 Gew.-% Cyclomethicon;
1-5 Gew.-% eines Tensids ausgewählt aus polyoxyethyliertem Fettsäure-(10-16C)Ester von Sorbitan (z. B. Span 20, Span 80); (8-16C)Fettsäureester und Polyoxypropylen / Polyoxyethylenblock-Copolymere (Poloxamers®);
1-8 Gew.-% von Crospovidon, Natriumcroscarmellose und/oder Trihydroxystearin; und
1-3 Gew.-% eines zweiwertigen Calcium- und/oder Magnesiumsalzes.

13. Pharmazeutische Zusammensetzung nach einem der Ansprüche 1 bis 12 zur Verwendung bei der Behandlung von topischen Infektionen, wahlweise wobei die topische Infektion ausgewählt ist aus Impetigo, MRSA oder einer komplizierten Haut- und/oder Strukturinfektion (cSSSI).

14. Pharmazeutische Zusammensetzung nach einem der Ansprüche 1 bis 12 zur Verwendung bei der Beseitigung von topischen MRSA vor einer Operation.

15. Verfahren zum Zubereiten einer pharmazeutischen Zusammensetzung nach Anspruch 1, wobei das Verfahren das Suspendieren von Daptomycin oder einem pharmazeutisch unbedenklichen Salz davon in einem Ölvehikel umfasst.

16. Vorrichtung, die eine pharmazeutische Zusammensetzung nach einem der Ansprüche 1 bis 12 umfasst, wobei die Vorrichtung dazu konfiguriert ist, ein Volumen der Zusammensetzung für topische Anwendung auszugeben.

## Revendications

1. Composition pharmaceutique convenant à l'application topique sur le corps, ladite composition comprenant de la daptomycine ou un sel de celle-ci de qualité pharmaceutique qui est dissous et/ou suspendu dans un véhicule huileux, la daptomycine ou son sel de qualité pharmaceutique étant présent à raison de 0,01 % en poids à 10 % en poids de la composition totale, et la teneur en humidité de la composition étant inférieure à 1 % en poids.

2. Composition pharmaceutique selon la revendication 1, dans laquelle :
(a) la daptomycine ou son sel de qualité pharmaceutique est uniformément dispersé ou dispersable dans le véhicule huileux ; et/ou
(b) la daptomycine ou son sel de qualité pharmaceutique est présent sous forme de particules discrètes, les particules ayant optionnellement un D90 de 75 µm ou moins.

3. Composition pharmaceutique selon l'une quelconque des revendications précédentes, dans laquelle le véhicule huileux :
(a) comprend un ou plusieurs constituants lipidiques sélectionnés dans le groupe constitué de :
mono-, di- et/ou triglycérides comprenant glycérol et acides gras (6-18C) ;
esters de propylène glycol d'acides gras (6-18C) ;
esters d'acides gras (6-18C) ;
alcools gras (6-18C) ;
fluides/huiles de silicone ; et
lanoline/dérivés de lanoline ; et/ou
(b) constitue entre 70 % en poids et 99,5 % en poids de la composition ; et/ou
(c) comprend un émollient huileux et ledit émollient constitue jusqu'à 70 % en poids de la composition ; et/ou
(d) comprend un ou plusieurs constituants lipidiques sélectionnés dans le groupe constitué de :
mono-, di- et/ou triglycérides comprenant glycérol et acides gras (6-18C) ;
esters de propylène glycol d'acides gras (6-18C) ;
esters d'acides gras (6-18C) (par ex. myristate d'isopropyle) ; et
fluides/huiles de silicone ; et/ou
(e) comprend :
5-45 % en poids d'esters d'acides gras (6-18C) ;
5-60 % en poids de mono-, di- et/ou triglycérides comprenant glycérol et acides gras (6-18C) ;
0-10 % en poids d'esters de propylène glycol d'acides gras (6-18C) ;
0-70 % en poids de fluides/huiles de silicone ; et
13-5 % en poids de lanoline/dérivé de lanoline.

4. Composition pharmaceutique selon l'une quelconque des revendications précédentes, dans laquelle le véhicule huileux de la composition comprend en outre un agent de suspension.

5. Composition pharmaceutique selon la revendication 4, dans laquelle l'agent de suspension :
(a) est sélectionné entre un ou plusieurs des suivants :
polymères réticulés judicieusement sous forme micronisée, par ex. PVP (crospovidone) et
croscarmellose sodique ;
argiles : phyllosilicate d'aluminium (bentonite) d'argiles micronisées ;
silicates de magnésium/aluminium ;
silices: silice (par ex. Syliod®, Cab-o-sil®), dioxyde de silicium (par ex. Aerosil®) ; et
huiles hydrogénées : par ex. trihydroxystéarine (Thixcin® R) ; et/ou
(b) est la crospovidone ; et/ou
(c) est présent à raison de 0,2 - 15 % en poids de la composition.

6. Composition pharmaceutique selon l'une quelconque des revendications précédentes, dans laquelle le véhicule huileux comprend en outre un ou plusieurs surfactants huileux.

7. Composition pharmaceutique selon la revendication 6, dans laquelle le surfactant huileux :
(a) est présent en quantité jusqu'à 7,5 % en poids de la composition totale ; et/ou
(b) a un HLB (rapport hydrophile-lipophile) inférieur à 7 ; et/ou
(c) est sélectionné dans le groupe constitué de : esters d'acides gras (10-18C) polyoxyéthylés de sorbitan (par ex. Span 20, Span 80) ; esters d'acides gras (8-18C), et copolymères séquencés de polyoxypropylène / polyoxyéthylène (Poloxamers®).

8. Composition pharmaceutique selon l'une quelconque des revendications précédentes, la composition pharmaceutique comprenant un agent de suspension selon la revendication 4 ou 5 et un surfactant huileux selon la revendication 6 ou 7.

9. Composition pharmaceutique selon l'une quelconque des revendications précédentes, la composition comprenant en outre un stabilisateur de sel métallique bivalent, optionnellement sélectionné entre : sels d'acides gras de calcium et/ou de magnésium (par ex. stéarate de calcium et/ou de magnésium), sulfate de calcium et/ou de magnésium, trisilicate de calcium et/ou de magnésium, et carbonate de calcium et/ou de magnésium, optionnellement où la quantité de stabilisateur présent est de 0 - 5 % en poids de la composition totale.

10. Composition pharmaceutique selon l'une quelconque des revendications précédentes, la composition comprenant :
(i) 0,05 - 5 % en poids de daptomycine ou d'un sel de celle-ci de qualité pharmaceutique ;
(ii) 75 - 99,5 % en poids d'un véhicule huileux ; et
(iii) optionnellement 0,5 - 10 % en poids d'un agent de suspension.

11. Composition pharmaceutique selon l'une quelconque des revendications précédentes, la composition comprenant :
0,05 - 5 % en poids de daptomycine ou d'un sel de celle-ci de qualité pharmaceutique ;
5-45 % en poids d'esters d'acides gras (6-18C) (par ex. myristate d'isopropyle) ;
5-60 % en poids de mono-, di- et/ou triglycérides comprenant glycérol et acides gras (6-18C) ;
0-10 % en poids d'esters de propylène glycol d'acides gras (6-18C) ;
0-70 % en poids d'huiles de silicone (telles que, par exemple, diméthicone, hexaméthyldisiloxane, octaméthyltrisiloxane et cyclométhicone);
0-5 % en poids de lanoline/dérivés de lanoline ;
jusqu'à 7,5 % en poids d'un surfactant sélectionné entre : esters d'acides gras (10-18C) polyoxyéthylés de sorbitan (par ex. Span 20, Span 80) ; esters d'acides gras (8-18C), et copolymères séquencés de polyoxypropylène / polyoxyéthylène (Poloxamers®) ;
0,5 - 10 % en poids d'un agent de suspension sélectionné entre un ou plusieurs des suivants :
polymères réticulés judicieusement sous forme micronisée, par ex. e.g PVP (crospovidone) & croscarmellose sodique ;
argiles : phyllosilicate d'aluminium d'argiles micronisées (bentonite);
silicates de magnésium/aluminium ;
silices : silice (Syliod®, Cab-o-sil®), dioxyde de silicium (Aerosil®) ; et
huiles hydrogénées : trihydroxystéarine (Thixcin® R) ; et
0-5 % en poids d'un sel métallique bivalent.

12. Composition pharmaceutique selon l'une quelconque des revendications précédentes, la composition comprenant :
0,2 - 4 % en poids de daptomycine ou d'un sel de celle-ci de qualité pharmaceutique ;
10 - 40 % de myristate d'isopropyle ;
10-60 % en poids de triglycéride caprylique/caprique / triglycéride à chaîne moyenne ;
0-30 % en poids de diméthicone ;
0-40 % en poids d'hexaméthyldisiloxane ;
0-30 % en poids de cyclométhicone ;
1-5 % en poids d'un surfactant sélectionné entre : esters d'acides gras (10-16C) polyoxyéthylés de sorbitan (par ex. Span 20, Span 80) ; esters d'acides gras (8-16C), et copolymères séquencés de polyoxypropylène / polyoxyéthylène (Poloxamers®) ;
1-8 % en poids de crospovidone, croscarmellose sodique et/ou trihydroxystéarine ; et
1-3 % en poids d'un sel bivalent de calcium et/ou magnésium.

13. Composition pharmaceutique selon l'une quelconque des revendications 1 à 12 pour utilisation dans le traitement d'infections topiques, ladite infection topique étant optionnellement sélectionnée entre : impétigo, SARM ou une infection compliquée de la peau et/ou de structure de la peau (IcPSP).

14. Composition pharmaceutique selon l'une quelconque des revendications 1 à 12 pour utilisation dans l'éradication du SARM topique avant une intervention chirurgicale.

15. Procédé de préparation d'une composition pharmaceutique selon la revendication 1, ledit procédé comprenant la suspension de daptomycine ou d'un sel de celle-ci de qualité pharmaceutique dans un véhicule huileux.

16. Dispositif comprenant une composition pharmaceutique selon l'une quelconque des revendications 1 à 12, le dispositif étant configuré pour administrer un volume de la composition pour application topique.
